(19) Europäisches Patentamt
European Patent Office
Office européen des brevets

(11) Numéro de publication : **0 130 877**
**B1**

(12) # FASCICULE DE BREVET EUROPÉEN

(45) Date de publication du fascicule du brevet : **28.09.88**

(51) Int. Cl.⁴ : **C 07 B 39/00**// C07C25/02, C07C25/13, C07C63/70, C07C39/27, C07C79/12, C07C87/60, C07C49/80, C07C43/225, C07C25/22, C07C65/05, C07C149/34, C08G61/02

(21) Numéro de dépôt : **84401232.8**

(22) Date de dépôt : **15.06.84**

(54) **Procédé de chloration de dérivés aromatiques.**

(30) Priorité : **23.06.83 FR 8310373**

(43) Date de publication de la demande : **09.01.85 Bulletin 85/02**

(45) Mention de la délivrance du brevet : **28.09.88 Bulletin 88/39**

(84) Etats contractants désignés : **AT BE CH DE FR GB IT LI LU NL SE**

(56) Documents cités : **US-A- 2 881 224**
**Le dossier contient des informations techniques présentées postérieurement au dépôt de la demande et ne figurant pas dans le présent fascicule.**

(73) Titulaire : **RHONE-POULENC CHIMIE**
**25, quai Paul Doumer**
**F-92408 Courbevoie Cédex (FR)**

(72) Inventeur : **Desbois, Michel**
**526 Chemin du Bois**
**F-69140 Rillieux (FR)**
Inventeur : **Disdier, Camille**
**54 Avenue Cabias**
**F-69004 Lyon (FR)**

(74) Mandataire : **Le Pennec, Magali et al**
**RHONE-POULENC INTERSERVICES Service Brevets Chimie 25, Quai Paul Doumer**
**F-92408 Courbevoie Cédex (FR)**

**EP 0 130 877 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé de chloration de dérivés aromatiques.

Elle concerne plus particulièrement un procédé de chloration de dérivés aromatiques par action du chlore gazeux.

Il est connu depuis longtemps de fixer du chlore sur un noyau aromatique à basse température et par voie catalytique à l'aide de chlorure ferrique. L'un des inconvénients majeurs de ce procédé est que le catalyseur n'est pas recyclable et en plus son élimination pose des problèmes de nuisance et de pollution importants. Vu les efforts actuels de protection de l'environnement et les projets de normes antipollution, on cherche depuis longtemps un procédé de chloration évitant l'utilisation de chlorure ferrique dont l'homme de l'art connaît les effets nocifs sur l'environnement.

Un autre inconvénient de l'utilisation de $FeCl_3$ comme catalyseur est le fait que ce produit est extrêmement difficile à séparer ou à détruire en fin de réaction.

D'autre part, dans le cas tout particulier de chloration de composés trifluorométhylés aromatiques à l'aide de chlorure ferrique apparaissent des phénomènes de défluoration diminuant le rendement. On a donc essayé de s'affranchir de ce catalyseur à base de $FeCl_3$.

On connaît ainsi dans l'art antérieur le brevet allemand n° 1034609 qui décrit un procédé de chloration de dérivés aromatiques à l'aide d'un catalyseur constitué de platine et d'un halogénure métallique tel que par exemple $PdCl_3$ fixé sur un support constitué d'alumine.

Il est aussi décrit dans le brevet allemand n° 825397 un procédé permettant de chlorer des dérivés aromatiques à l'aide d'un catalyseur constitué de trifluorure de bore.

On connait encore le brevet US-A-2 881 224 qui décrit la chloration de dérivés aromatiques en présence de $BF_3$ dans de l'acide fluorhydrique.

Contrairement à un préjugé technique depuis longtemps établi conduisant l'homme de l'art à considérer que la chloration des dérivés aromatiques ne pouvait se faire que par voie catalytique, on a découvert qu'il est possible d'opérer cette chloration sans catalyseur.

En effet, la présente invention a pour objet un procédé de chloration de dérivés aromatiques caractérisé en ce qu'on fait réagir le dérivé aromatique avec du chlore gazeux dans de l'acide fluorhydrique liquide, en l'absence de catalyseur.

Le présent procédé peut être utilisé pour chlorer tous les dérivés aromatiques quels que soient les substituants présents sur le noyau. On peut mettre en œuvre notamment les dérivés benzéniques et les dérivés polycycliques comme ceux du naphtalène et de l'anthracène.

La position de fixation du chlore sur le noyau se fera selon les règles de substitution biens connues de l'homme de l'art, en fonction de la présence d'autres radicaux ortho, para ou méta orienteurs.

L'acide fluorhydrique utilisé pour la présente invention est de préférence anhydre. Le rapport molaire de l'acide fluorhydrique au composé aromatique de départ est de préférence compris entre 5 et 50.

La quantité de chlore mise en œuvre est fixée par l'homme de l'art compte-tenu du produit recherché correspondant à une mono ou polychloration. Pour une monochloration, on opère de préférence en présence d'un défaut stoechiométrique de chlore, c'est-à-dire avec un rapport molaire chlore au composé aromatique compris de préférence entre 0,5 et 0,9. Pour une polychloration, on préfère opérer avec un excès de chlore. Le chlore peut être mis en œuvre dans une enceinte fermée sous pression autogène (généralement 1 à 50 bars) ou sous pression atmosphérique par barbotage ou dans tout autre dispositif connu de l'homme de l'art.

La température de mise en œuvre est comprise de préférence entre — 20 °C et 150 °C.

Lorsque la température sera supérieure à 20 °C, la réaction devra avoir lieu sous pression puisque l'acide fluorhydrique doit être liquide.

La durée de réaction varie de quelques minutes à quelques heures.

Cette durée de réaction varie avec le nombre d'atomes de chlore que l'on veut fixer sur le noyau ainsi qu'avec les matières premières de départ et avec la température de la réaction.

Le produit final chloré aromatique est isolé par exemple par distillation de l'acide fluorhydrique qui ainsi est récupérable et recyclable, ce qui est un avantage important du procédé de l'invention.

Il peut aussi être isolé par extraction à l'aide de solvants organiques bien connus de l'homme de l'art.

L'invention trouve une application particulièrement intéressante dans la chloration des dérivés aromatiques : perfluoroalkylés, perfluoroalkoxylés, perfluorothioalkylés comme par exemple les dérivés aromatiques trifluorométhylés ($-CF_3$), trifluorométhoxylés ($-OCF_3$), trifluorothiométhylés ($-SCF_3$). Ces dérivés chlorés sont utilisés dans l'industrie pharmaceutique et phytosanitaire.

On peut citer comme exemples de produits pouvant être mis en œuvre selon le procédé de la présente invention : le trifluorométhylbenzène, le chlorure de benzoyle, le phénol, le nitrobenzène, le p-chlorotrifluorométhylbenzène, le p-fluoronitrobenzène, le dichloro-2-4 phénol, l'o-nitrotoluène, le chloro-benzène, le fluoro-benzène, l'acide benzoïque, le m-bistrifluorométhylbenzène, l'aniline, l'acétophénone, la benzophénone, l'anisole, le toluène, l'anthracène, l'oxyde de diphényle, l'o-crésol, l'acide salicylique, le trifluorométhoxybenzène, le trifluorométhylthiobenzène.

La présente invention sera plus aisément comprise à l'aide des exemples suivants.

Exemple 1

Dans un réacteur de 250 ml, agité par un barreau aimanté et refroidi aux environs de 0 °C, on introduit 100 ml (5 m) d'acide fluorhydrique anhydre et 7,8 g (0,1 m) de benzène. Le réacteur est fermé et porté à la pression de 4 bars (à 0 °C) par du chlore gazeux.

On chauffe alors le tout, sous agitation, à 27 °C pendant 3 heures 45 minutes. Après refroidissement, de nouveau vers 0 °C, le réacteur est décomprimé et le brut réactionnel obtenu coulé sur 110 g de glace pilée. Le mélange hétérogène, issu de ce traitement, est extrait par 3 fois 100 $cm^3$ de chlorure de méthylène. Après décantations, les phases organiques sont rassemblées. L'ensemble est lavé par 2 fois 100 $cm^3$ d'eau permutée et séché. Les analyses effectuées par chromatographie en phase gazeuse (% aire) spectrométrie IR et spectrométrie de masse nous donnent le résultat suivant :

| | |
|---|---|
| Benzène | 10,3 % |
| Monochlorobenzène | 77,6 % |
| p-dichlorobenzène | 7,6 % |
| o-dichlorobenzène | 4,5 % |

Exemple 2

On procède de manière identique à l'exemple 1 avec les composés et conditions ci-dessous et en remplaçant le traitement du brut réactionnel à la glace par une extraction de ce brut à l'aide de 2 fois 100 $cm^3$ de tétrachlorure de carbone, le traitement de ces phases organiques étant poursuivi normalement.

| | |
|---|---|
| Acide fluorhydrique anhydre | 100 g (5 m) |
| Trifluorométhylbenzène | 29,2 g (0,2 m) |
| Température | 80 °C |
| Pression de chlore | 5 bars à 20 °C |
| Durée | 4 heures |

Les analyses effectuées par chromatographie en phase gazeuse (% aire), spectrométrie IR et spectrométrie de masse nous donnent le résultat suivant :

| | |
|---|---|
| Trifluorométhylbenzène | 59 % |
| o-chlorotrifluorométhylbenzène | 2 % |
| m-chlorotrifluorométhylbenzène | 28 % |
| p-chlorotrifluorométhylbenzène | 5 % |
| polychlorotrifluorométhylbenzènes | 6 % |

Exemple 3

On procède de manière identique à l'exemple 1 avec les composés et conditions suivantes :

| | |
|---|---|
| Acide fluorhydrique anhydre | 50 g (2,5 m) |
| Chlorure de benzoyle | 14,1 g (0,1 m) |
| Température | 80 °C |
| Pression de chlore | 6 bars à 20 °C |
| Durée | 4 heures |

Les analyses effectuées par chromatographie en phase gazeuse (% aire), spectrométrie IR et spectrométrie de masse nous donnent le résultat suivant :

| | |
|---|---|
| Fluorure de benzoyle | 78,3 % |
| Fluorure de m-chlorobenzoyle | 16,8 % |
| Fluorures d'o-, p- et polychlorobenzoyle | 4,9 % |

Exemple 4

On procède de manière identique à l'exemple 1 avec les composés et conditions ci-dessous et en remplaçant le traitement du brut réactionnel à la glace par une distillation jusqu'à 80 °C en pied sous pression atmosphérique de ce brut afin d'éliminer au maximum l'acide fluorhydrique solvant :

| | |
|---|---|
| Acide fluorhydrique anhydre | 60 g (3 m) |
| Phénol | 9,5 g (0,1 m) |
| Température | environ — 20 °C |
| Pression de chlore | 4 bars à — 30 °C |

| | |
|---|---|
| Durée | 3 heures 15 |

Les analyses effectuées par chromatographie en phase gazeuse (% aire), spectrométrie IR et spectrométrie de masse nous donnent le résultat suivant :

| | |
|---|---|
| Phénol | 48,7 % |
| p-chlorophénol | 37 % |
| o-chlorophénol | 7 % |
| polychlorophénols | 17,3 % |

Exemple 5

On procède de manière identique à l'exemple 1 avec les composés et conditions suivants :

| | |
|---|---|
| Acide fluorhydrique anhydre | 50 g (2,5 m) |
| Nitrobenzène | 12,3 g (0,1 m) |
| Température | 100 °C |
| Pression de chlore | 5 bars à 20 °C |
| Durée | 23 heures |

Les analyses effectuées par chromatographie en phase gazeuse (% aire), spectrométrie IR et spectrométrie de masse nous donnent le résultat suivant :

| | |
|---|---|
| Nitrobenzène | 61,5 % |
| m-chloronitrobenzène | 26,6 % |
| autres chloronitrobenzènes | 11,9 % |

Exemple 6

On procède de manière identique à l'exemple 1 avec les composés et conditions suivants :

| | |
|---|---|
| Acide fluorhydrique anhydre | 100 g (5 m) |
| p-chlorotrifluorométhylbenzène | 18 g (0,1 m) |
| Température | 80 °C |
| Pression de chlore | 5 bars à 20 °C |
| Durée | 22 heures 20 |

Les analyses effectuées par chromatographie en phase gazeuse (% aire), spectrométrie IR et spectrométrie de masse nous donnent le résultat suivant :

| | |
|---|---|
| p-chlorotrifluorométhylbenzène | 28 % |
| dichloro-3,4 trifluorométhylbenzène | 69 % |
| autres chlorotrifluorométhylbenzène | 3 % |

Exemple 7

On procède de manière identique à l'exemple 1 avec les composés et conditions suivants :

| | |
|---|---|
| Acide fluorhydrique anhydre | 100 g (5 m) |
| p-fluoronitrobenzène | 14,1 g (0,1 m) |
| Température | 100 °C |
| Pression de chlore | 5 bars à 20 °C |
| Durée | 5 heures 40 |

Les analyses effectuées par chromatographie en phase gazeuse (% aire), spectrométrie IR et spectrométrie de masse nous donnent le résultat suivant :

| | |
|---|---|
| p-fluoronitrobenzène | 97,1 % |
| Fluoro-4 chloro-3 nitrobenzène | 2,9 % |

Exemple 8

On procède de manière identique à l'exemple 1 avec les composés et conditions suivants :

| | |
|---|---|
| Acide fluorhydrique anhydre | 100 g (5 m) |

| | |
|---|---|
| Dichloro-2,4 phénol | 16,3 g (0,1 m) |
| Température | 100 °C |
| Pression de chlore | 6 bars à 20 °C |
| Durée | 18 heures 45 |

Les analyses effectuées par chromatographie en phase gazeuse (% aire), spectrométrie IR et spectrométrie de masse nous donnent le résultat suivant :

| | |
|---|---|
| Dichloro-2,4 phénol | 22 % |
| Trichlorophénol | 73 % |
| Tétrachlorophénol | 1 % |

Exemple 9

On procède de manière identique à l'exemple 1 avec les composés et conditions suivants :

| | |
|---|---|
| Acide fluorhydrique anhydre | 100 g (5 m) |
| o-nitrotoluène | 27,4 g (0,2 m) |
| Température | 20 °C |
| Pression de chlore | 5 bars à 20 °C |
| Durée | 4 heures |

Les analyses effectuées par chromatographie en phase gazeuse (% aire), spectrométrie de masse nous donnent le résultat suivant :

| | |
|---|---|
| o-nitrotoluène | 96 % |
| Chloro-o-nitrotoluène | 4 % |

Exemple 10

On procède de manière identique à l'exemple 1 avec les composés et conditions suivants :

| | |
|---|---|
| Acide fluorhydrique anhydre | 100 g (5 m) |
| Chlorobenzène | 22,5 g (0,2 m) |
| Température | 20 °C |
| Pression de chlore | 5 bars à 20 °C |
| Durée | 4 heures |

Les analyses effectuées par chromatographie en phase gazeuse (% aire), spectrométrie IR et spectrométrie de masse nous donnent le résultat suivant :

| | |
|---|---|
| Chlorobenzène | 82 % |
| p-dichlorobenzène | 13 % |
| o-dichlorobenzène | 5 % |

Exemple 11

On procède de manière identique à l'exemple 1 avec les composés et conditions suivants :

| | |
|---|---|
| Acide fluorhydrique anhydre | 100 g (5 m) |
| Fluorobenzène | 9,6 g (0,1 m) |
| Température | 20 °C |
| Pression de chlore | 5 bars à 20 °C |
| Durée | 20 heures |

Les analyses effectuées par chromatographie en phase gazeuse (% aire), spectrométrie IR et spectrométrie de masse nous donnent le résultat suivant :

| | |
|---|---|
| Fluorobenzène | 0 % |
| p-chlorofluorobenzène | 76,5 % |
| o-chlorofluorobenzène | 1,3 % |
| Dichlorofluorobenzènes | 21,4 % |
| Trichlorofluorobenzènes | 0,8 % |

Exemple 12

On procède de manière identique à l'exemple 1 avec les composés et conditions suivants :

| | |
|---|---|
| Acide fluorhydrique anhydre | 100 g (5 m) |
| Acide benzoïque | 12,2 g (0,1 m) |
| Température | 100 °C |
| Pression de chlore | 5 bars à 20 °C |
| Durée | 23 heures 45 |

Les analyses effectuées par chromatographie en phase gazeuse (% aire), spectrométrie IR et spectrométrie de masse nous donnent le résultat suivant :

| | |
|---|---|
| Acide benzoïque | 29 % |
| Acide m-chlorobenzoïque | 71 % |

## Exemple 13

On procède de manière identique à l'exemple 1 avec les composés et conditions suivants :

| | |
|---|---|
| Acide fluorhydrique anhydre | 100 g (5 m) |
| m-bistrifluorométhylbenzène | 21,4 g (0,1 m) |
| Température | 80 °C |
| Pression de chlore | 5 bars à 20 °C |
| Durée | 18 heures |

Les analyses effectuées par chromatrographie en phase gazeuse (% aire), spectrométrie IR et spectrométrie de masse nous donnent le résultat suivant :

| | |
|---|---|
| m-bistrifluorométhylbenzène | 97,7 % |
| Chloro m-bistrifluorométhylbenzène | 2,3 % |

## Exemple 14

On procède de manière identique à l'exemple 1 avec les composés et conditions suivants :

| | |
|---|---|
| Acide fluorhydrique anhydre | 100 g (5 m) |
| Aniline | 9,3 g (0,1 m) |
| Température | 120 °C |
| Pression de chlore | 5 bars à 20 °C |
| Durée | 18 heures |

Les analyses effectuées par chromatographie en phase gazeuse (% aire), spectrométrie IR et spectrométrie de masse nous donnent le résultat suivant :

| | |
|---|---|
| Aniline | 78 % |
| m et/ou p-chloroaniline | 6 % |
| o-chloroaniline | 0,8 % |
| Polychloroaniline | 15,2 % |

## Exemple 15

On procède de manière identique à l'exemple 1 avec les composés et conditions suivants :

| | |
|---|---|
| Acide fluorhydrique anhydre | 100 g (5 m) |
| Acétophénone | 12 g (0,1 m) |
| Température | 80 °C |
| Pression de chlore | 4 bars à 20 °C |
| Durée | 18 heures |

Les analyses effectuées par chromatographie en phase gazeuse (% aire), spectrométrie IR et spectrométrie de masse nous donnent le résultat suivant :

| | |
|---|---|
| Acétophénone | 60 % |
| di et trichlorométhylphénylcétone | 30,1 % |
| Chloroacétophénones | 7 % |

Exemple 16

On procède de manière identique à l'exemple 1 avec les composés et conditions suivants :

| | |
|---|---|
| Acide fluorhydrique anhydre | 100 g (5 m) |
| Benzophénone | 18,2 g (0,1 m) |
| Température | 80 °C |
| Pression de chlore | 4 bars à 20 °C |
| Durée | 27 heures |

Les analyses effectuées par chromatographie en phase gazeuse (% aire), spectrométrie IR et spectrométrie de masse nous donnent le résultat suivant :

| | |
|---|---|
| Benzophénone | 43 % |
| Monochlorobenzophénones | 20 % |
| Polychlorobenzophénones | 37 % |

Exemple 17

On procède de manière identique à l'exemple 1 avec les composés et conditions suivants :

| | |
|---|---|
| Acide fluorhydrique anhydre | 100 g (5 m) |
| Aniline | 9,3 g (0,1 m) |
| Température | 25 °C |
| Pression de chlore | 4 bars à 0 °C |
| Durée | 19 heures |

Les analyses effectuées par chromatographie en phase gazeuse (% aire), spectrométrie IR et spectrométrie de masse nous donnent le résultat suivant :

| | |
|---|---|
| Aniline | 96 % |
| p et/ou m-chloroaniline | 2 % |
| o-chloroaniline | 1 % |
| Dichloroaniline | 1 % |

Exemple 18

On procède de manière identique à l'exemple 1 avec les composés et conditions suivants :

| | |
|---|---|
| Acide fluorhydrique anhydre | 100 g (5 m) |
| Anisole | 10,8 g (0,1 m) |
| Température | 20 °C |
| Pression de chlore | 4 bars à 20 °C |
| Durée | 2 heures 30 |

Les analyses effectuées par chromatographie en phase gazeuse (% aire), spectrométrie IR et spectrométrie de masse nous donnent le résultat suivant :

| | |
|---|---|
| Anisole | 5 % |
| p-chloroanisole | 43 % |
| o-chloroanisole | 25,5 % |
| Polychloroanisoles | 26,5 % |

Exemple 19

On procède de manière identique à l'exemple 1 avec les composés et conditions suivants :

| | |
|---|---|
| Acide fluorhydrique | 50 g (2,5 m) |
| Toluène | 9,2 g (0,1 m) |
| Température | 10 °C |
| Pression de chlore | 4 bars à 10 °C |
| Durée | 4 heures 15 |

Les analyses effectuées par chromatographie en phase gazeuse (% aire), spectrométrie IR et spectrométrie de masse nous donnent le résultat suivant :

| | |
|---|---|
| Toluène | 14 % |
| Monochlorotoluène | 71 % |
| Polychlorotoluène | 15 % |

Exemple 20

On procède de manière identique à l'exemple 1 avec les composés et conditions suivants :

| | |
|---|---|
| Acide fluorhydrique anhydre | 100 (5 m) |
| Anthracène | 12,8 g (0,1 m) |
| Température | 20 °C |
| Pression de chlore | 4 bars à 20 °C |
| Durée | 4 heures 30 |

Les analyses effectuées par chromatographie en phase gazeuse (% aire), spectrométrie IR et spectrométrie de masse nous donnent le résultat suivant :

| | |
|---|---|
| Anthracène | 61 % |
| Monochloroanthracènes | 12 % |
| Polychloroanthracènes | 27 % |

Exemple 21

On procède de manière identique à l'exemple 1 avec les composés suivants :

| | |
|---|---|
| Acide fluorhydrique anhydre | 20 g (1 m) |
| Diphényléther | 17 g (0,1 m) |
| Température | 20 °C |
| Pression de chlore | 2 bars à 20 °C |
| Durée | 19 heures 30 |

Les analyses effectuées par chromatographie en phase gazeuse (% aire), spectrométrie IR et spectrométrie de masse nous donnent le résultat suivant :

| | |
|---|---|
| Diphényléther | 62 % |
| Monochlorodiphényléther | 25,3 % |
| Polychlorodiphényléther | 12,7 % |

Exemple 22

On procède de manière identique à l'exemple 1 avec les composés et conditions suivants :

| | |
|---|---|
| Acide fluorhydrique anhydre | 50 g (2,5 m) |
| o-crésol | 10,8 g (0,1 m) |
| Température | 10 °C |
| Pression de chlore | 4 bars à 10 °C |
| Durée | 19 heures 15 |

Les analyses effectuées par chromatographie en phase gazeuse (% aire), spectrométrie IR et spectrométrie de masse nous donnent le résultat suivant :

| | |
|---|---|
| o-crésol | 66,7 % |
| Chloro-4 méthyl-2 phénol | 18,2 % |
| Autres chloro-o-crésol | 15,1 % |

Exemple 23

On procède de manière identique à l'exemple 1 avec les composés et conditions suivants :

| | |
|---|---|
| Acide fluorhydrique anhydre | 100 g (5 m) |
| Acide salicylique | 13,8 g (0,1 m) |
| Température | 80 °C |
| Pression de chlore | 5 bars à 20 °C |
| Durée | 23 heures 20 |

Les analyses effectuées par chromatographie en phase gazeuse (% aire), spectrométrie IR et spectrométrie de masse nous donnent le résultat suivant :

| | |
|---|---|
| Acide salicylique | 0 % |
| Acide chloro-5 hydroxy-2 benzoïque | 68,5 % |
| Acide chloro-3 hydroxy-2 benzoïque | 10 % |
| Acide dichloro-3,5 hydroxy-2 benzoïque | 20,5 % |

Exemple 24

On procède de manière identique à l'exemple 1 avec les composés et conditions suivants :

| | |
|---|---|
| Acide fluorhydrique anhydre | 50 g (2,5 m) |
| Trifluorométhoxybenzène | 16,2 g (0,1 m) |
| Température | 50 °C |
| Pression de chlore | 4 bars à 20 °C |
| Durée | 5 heures 15 |

Les analyses effectuées par chromatographie en phase gazeuse (% aire), spectrométrie IR et spectrométrie de masse nous donnent le résultat suivant :

| | |
|---|---|
| Trifluorométhoxybenzène | 24 % |
| p-chlorotrifluorométhoxybenzène | 64,5 % |
| o-chlorotrifluorométhoxybenzène | 10 % |
| dichlorotrifluorométhoxybenzène | 1,5 % |

Exemple 25

On procède de manière identique à l'exemple 1 avec les composés et conditions suivants :

| | |
|---|---|
| Acide fluorhydrique anhydre | 25 g (1,25 m) |
| Trifluorométhylthiobenzène | 4,45 g (0,025 m) |
| Température | 50 °C |
| Pression de chlore | 4 bars à 20 °C |
| Durée | 5 heures 15 |

Les analyses effectuées par chromatographie en phase gazeuse (% aire), spectrométrie IR et spectrométrie de masse nous donnent le résultat suivant :

| | |
|---|---|
| Trifluorométhylthiobenzène | 0 % |
| Monochlorotrifluorométhylthiobenzène | 0,5 % |
| Dichlorotrifluorométhylthiobenzène | 4,2 % |
| Trichlorotrifluorométhylthiobenzène | 74 % |
| Autres chlorotrifluorométhylthiobenzène | 21,3 % |

Exemple 26

On procède de manière identique à l'exemple 1 mais à pression atmosphérique et introduction du chlore par bullage au sein du milieu réactionnel et avec les composés et conditions suivants :

| | |
|---|---|
| Acide fluorhydrique anhydre | 100 g (5 m) |
| Alcool benzylique | 10,8 g (0,1 m) |
| Température | 0 °C |
| Pression de chlore | P. atmosphérique |
| Durée | 1 heures 10 mm |

Les analyses effectuées par spectrométrie IR nous donnent le résultat suivant :
Présence d'un polymère chloré sur le noyau aromatique de structure :

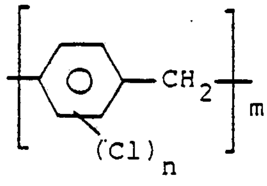

Exemple 27

On procède de manière identique à l'exemple 1 avec les composés et conditions suivants :

| | |
|---|---|
| Acide fluorhydrique anhydre | 50 g (2,5 m) |
| Chloro-2 trifluorométhyl-4 phénol | 9,8 g (0,05 m) |
| Température | 48 °C |
| Pression de chlore | 4 bars à 20 °C |
| Durée | 4 heures |

Les analyses effectuées par chromatographie en phase gazeuse (% aire), spectrométrie IR et spectrométrie de masse nous donnent le résultat suivant :

| | |
|---|---|
| Dichloro-2,6 et dichloro-2,5 trifluorométhyl-4 phénol | 88 % |
| Trichloro trifluorométhyl-4 phénol | 8 % |

**Revendications**

1. Procédé de chloration de dérivés aromatiques caractérisé en ce qu'on fait réagir le dérivé aromatique avec du chlore gazeux dans de l'acide fluorhydrique liquide en l'absence de catalyseur.
2. Procédé selon la revendication 1 caractérisé en ce que l'acide fluorhydrique est anhydre.
3. Procédé selon les revendications 1 ou 2 caractérisé en ce que l'acide fluorhydrique est utilisé selon un rapport molaire acide fluorhydrique au composé aromatique compris entre 5 et 50.
4. Procédé selon l'une quelconque des revendications précédentes caractérisé en ce que la chloration s'effectue entre — 20 °C et 150 °C.
5. Procédé selon l'une quelconque des revendications précédentes caractérisé en ce que la chloration s'effectue sous pression autogène.
6. Procédé selon l'une quelconque des revendications précédentes caractérisé en ce que la chloration s'effectue sous pression atmosphérique.
7. Procédé selon l'une quelconque des revendications précédentes caractérisé en ce que le dérivé aromatique est choisi parmi les dérivés perchloroalkyles, perchloroalkoxyles, perchlorothioalkyles.
8. Procédé selon la revendication 7 caractérisé en ce que le dérivé aromatique est choisi parmi les trichlorométhylbenzènes, trichlorométhoxybenzènes, trichlorométhylthiobenzènes.

**Claims**

1. Process for chlorination of aromatic derivatives, characterized in that the aromatic derivative is reacted with gaseous chlorine in liquid hydrofluoric acid in the absence of catalyst.
2. Process according to Claim 1, characterized in that the hydrofluoric acid is anhydrous.
3. Process according to Claims 1 or 2, characterized in that the hydrofluoric acid is employed in a molar ratio of hydrofluoric acid to the aromatic compound of between 5 and 50.
4. Process according to any one of the preceding claims, characterized in that the chlorination takes place between — 20 °C and 150 °C.
5. Process according to any one of the preceding claims, characterized in that the chlorination takes place under autogenous pressure.
6. Process according to any one of the preceding claims, characterized in that the chlorination takes place at atmospheric pressure.
7. Process according to any one of the preceding claims, characterized in that the aromatic derivative is chosen from perchloroalkyl, perchloroalkoxy and perchlorothioalkyl derivatives.
8. Process according to Claim 7, characterized in that the aromatic derivative is chosen from trichloromethylbenzenes, trichloromethoxybenzenes and trichloromethylthiobenzenes.

**Patentansprüche**

1. Verfahren zur Chlorierung aromatischer Verbindungen, dadurch gekennzeichnet, daß man die aromatische Verbindung mit gasförmigem Chlor in flüssiger Fluorwasserstoffsäure in Abwesenheit eines Katalysators reagieren läßt.
2. Verfahren nach Anspruch 1, dadurch gekennzeichnet, daß die Fluorwasserstoffsäure wasserfrei ist.
3. Verfahren nach den Ansprüchen 1 oder 2, dadurch gekennzeichnet, daß die Fluorwasserstoffsäure in einem molaren Verhältnis Fluorwasserstoffsäure zu aromatischer Verbindung zwischen 5 und 50 verwendet wird.

4. Verfahren nach einem der vorhergehenden Ansprüche, dadurch gekennzeichnet, daß die Chlorierung zwischen — 20 °C und 150 °C ausgeführt wird.

5. Verfahren nach einem der vorhergehenden Ansprüche, dadurch gekennzeichnet, daß die Chlorierung bei autogenem Druck durchgeführt wird.

6. Verfahren nach einem der vorhergehenden Ansprüche, dadurch gekennzeichnet, daß die Chlorierung bei atmosphärischem Druck durchgeführt wird.

7. Verfahren nach einem der vorhergehenden Ansprüche, dadurch gekennzeichnet, daß die aromatische Verbindung ausgewählt wird aus den Perchloralkyl-, Perchloralkoxyl-, Perchlorthioalkylverbindungen.

8. Verfahren nach Anspruch 7, dadurch gekennzeichnet, daß die aromatische Verbindung ausgewählt wird aus den Trichlormethylbenzolen, Trichlormethoxybenzolen, Trichlormethylthiobenzolen.